# EUROPEAN PATENT SPECIFICATION

(11) **EP 2 612 610 B1**
(45) Date of publication and mention of the grant of the patent: **11.05.2022**
(21) Application number: 11821704.1
(22) Date of filing: 29.08.2011
(51) Int. Cl.: A61F 9/013, A61F 9/007, A61B 17/3211, B24B 3/60, C25F 3/24

(54) **MEDICAL KNIFE**
MEDIZINISCHES MESSER
COUTEAU MÉDICAL

(30) Priority: 30.08.2010 JP 2010192068
(43) Date of publication of application: 10.07.2013
(73) Proprietor: Mani, Inc., Tochigi 321-3231 (JP)
(72) Inventor: SAITO, Masahiko, Utsunomiya-shi Tochigi 321-3231 (JP)
(74) Representative: Isarpatent
(86) International application number: PCT/JP2011/069389
(87) International publication number: WO 2012/029688

(56) References cited:
- WO-A1-2008/136410
- WO-A1-2008/136410
- JP-A- 7 289 559
- JP-A- 9 327 464
- JP-A- 2002 065 720
- US-A- 5 258 002
- US-A1- 2002 026 205
- US-A1- 2003 130 676
- US-A1- 2005 004 587
- US-A1- 2005 004 588
- US-A1- 2006 212 059
- US-A1- 2006 212 059
- US-A1- 2007 179 515

## Description

### [Technical Field]

The present invention relates to a medical knife used in surgery, such as a straight ophthalmic knife, an LRI knife for astigmatism correction, and the like.

### [Background Art]

The straight knife and the LRI knife used for ophthalmic surgery have a thin, sharp front end and a straight cutting edge several millimeters long extending from the end. When using them, mainly the front end and a cutting edge portion approximately 1 mm from the front end are shifted in a direction orthogonal to the axis of the knife and direction parallel to the cutting portion, so as to cut into a cornea or sclera.

The medical knife is conventionally made of martensitic stainless steel. Martensitic stainless steel can be quenched, and can thereby attain a desired hardness and favorable sharpness. US 2006/0212059 A1 discloses a knife having a blade tip configuration.

However, since martensitic stainless steel rusts easier than austenitic stainless steel, use of austenitic stainless steel that does not rust as easily for medical applications is desired. However, austenitic stainless steel wire cannot be quenched nor attain a predetermined hardness.

In response, an austenitic stainless steel wire rod is stretched and work hardened, so as to attain a predetermined hardness. The stainless steel wire that is stretched and work hardened as such has a crystalline structure spreading in strips along the length of the wire. This is referred to as a fibrous structure.

The method of manufacturing a medical knife from austenitic stainless steel having such a fibrous structure is almost the same as the case of manufacturing from martensitic stainless steel. That is, the medical knife is manufactured by cutting an austenitic stainless steel wire rod to a predetermined length to make a round bar, pressing an end of the round bar flat, molding the pressed portion into a knife shape through the following pressing step, and forming a cutting blade through grinding using a grinding stone or the like. In the martensitic case, it then undergoes heat treatment and final polishing, but in the austenitic case, it does not proceed past the heat treatment step.

The medical knife has a problem with sharpness, and therefore various measures have been taken to improve sharpness. Application of silicone is often performed as one of these measures. This is because a silicone film reduces frictional resistance (e.g., Patent Document 1).

A suture needle having grooves running almost orthogonal to the central axis thereof and grooves running along the length of the central axis is proposed in Patent Document 2. This is attained by polishing the raw material having a crystalline fibrous structure in a direction orthogonal to the length of the fibrous structure, making grooves running orthogonal to the length of the fibrous structure by abrasive grains, and then carrying out processing such as electrolytic polishing and chemical polishing, thereby etching the grooves by the abrasive grains so as to expose a part of the fibrous structure. The grooves running along the central axis result from performing electrolytic polishing on the pattern of the fibrous structure, the grooves running orthogonal to the central axis are made by abrasive grains during polishing. Silicone is applied to the suture needle that has vertical and horizontal grooves and then used. Such a structure allows improvement in adherence of silicone.

In addition, technology of electrolytically polishing a blade tip portion so as to remove burrs resulting from abrasive polishing, making the blade tip portion into a mirror-finished surface, finishing a sharp cutting blade, and improving sharpness is proposed in Patent Document 3.

### [Prior Art Documents]

### [Patent Documents]

[Patent Document 1] JP 2003-116866 A
[Patent Document 2] JP 3140508
[Patent Document 3] JP H08-238245 A

### [DISCLOSURE OF THE INVENTION]

### [Problem To Be Solved by the Invention]

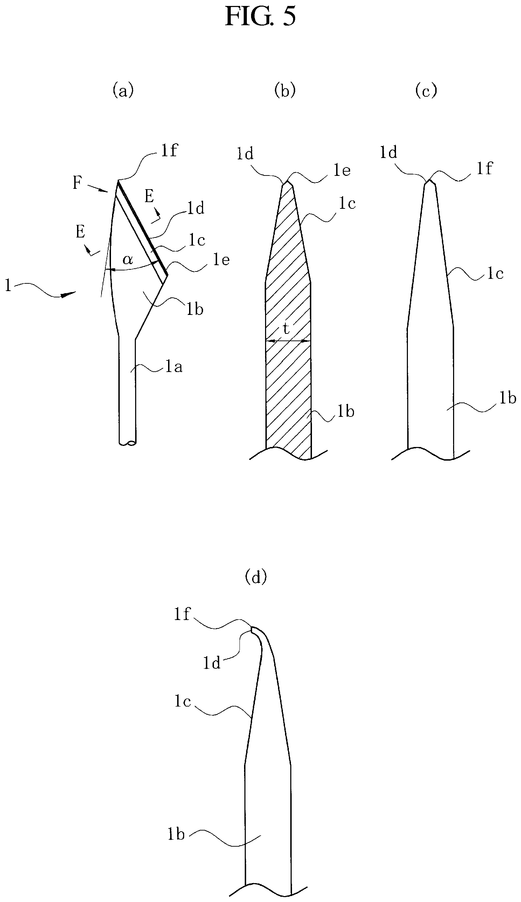

FIGS. 5 are diagrams illustrating a conventional straight knife, where FIG. 5(a) is a top view, FIG. 5(b) is a cross section of a cutting blade cut along a line E-E of FIG. 5(a), FIG. 5(c) is an enlarged view of a blade tip portion of the knife viewed from F of FIG. 5(a), and FIG. 5(d) is a diagram illustrating a bent blade tip.

A straight knife 1 shown in the diagrams is made of austenitic stainless steel. As described before, a stainless steel round bar 1a is cut to a predetermined length, a front end thereof is pressed flat into a planar cutting portion 1b, a side of the cutting portion 1b is ground from either side to form a slanted surface 1c, and a linear cutting blade 1d and an edge 1e are formed at the end of the slanted surface 1c.

The straight knife 1 generally has a small angle α of a blade tip If and thin thickness t as shown in FIG. 5(a) so as to improve sharpness. As a result, there is a problem that when forming an incision in a cornea, a sclera, or the like during ophthalmic surgery, the blade tip If of the austenitic stainless steel knife is easily bent. FIG. 5(d) is a diagram illustrating peripheral parts of the same blade tip If (referred to as 'blade tip portion' hereafter) as in FIG. 5(c), illustrating a bent state due to elasticity when cutting a cornea, a sclera, or the like. If the blade tip portion easily bends, it is difficult to use as a knife. Moreover, there is a problem that if deformation is great, it does not return to its original shape and cannot be used as a knife thereafter.

The present invention aims to solve the above problems, and to provide a medical knife made of austenitic stainless steel that can increase strength of a thin and sharp blade tip portion, is strong against deformation, and can prevent decrease in sharpness.

### [Solution to the Problem]

A medical knife of the present invention for achieving the above object is claimed in claim 1. Further embodiments of the invention are defined by the dependent claims.

Note that 'a blade tip of the knife is rounded' here includes a convex rounded surface on the whole not only having a circular arc with a fixed curvature radius, but also an elliptic arc, a parabola, and some irregularities.

A second medical knife of the present disclosure for achieving the above object is characterized by including a planar cutting portion and a slanted surface formed along the periphery of at least a part of the cutting portion. The slanted surface is electrolytically polished or chemically polished so as to form a cutting blade at a front end portion of the slanted surface, and the cutting blade is gradually curved in a centrally protruding form.

A third medical knife of the present disclosure for achieving the above object is characterized by including a planar cutting portion and a slanted surface formed along the periphery of at least a part of the cutting portion. The slanted surface is electrolytically polished or chemically polished so as to form a cutting blade constituted by a convex rounded surface at a front end portion of the slanted surface.

A combined configuration of any two of the first, the second and the third structure, or a combined configuration of all of the first, the second, and the third structures may be provided. Alternatively, a configuration where the electrolytic polishing or chemical polishing is applied to the entire length of the cutting blade, a configuration where the cutting blade is formed on two sides of the cutting portion adjacent to each other, or a configuration formed by a wrapping film may be provided.

### [Advantageous Effect of the Invention]

According to the medical knife of the present invention, since the blade tip is rounded through electrolytic polishing or chemical polishing, the blade tip portions is thick and difficult to bend, thereby allowing prevention of bending during use even if austenitic stainless steel is used. This brings about a beneficial effect of easy usability. Moreover, since austenitic stainless steel is used, a medical knife that does not rust as easily can be provided.

It seems that sharpness of the knife decreases when the blade tip is rounded. However, since it is rounded through electrolytic polishing or chemical polishing, the blade tip is the very small portion that is rounded. Furthermore, the burrs generated through grinding when forming the cutting blade are removed through electrolytic polishing or chemical polishing, thereby improving sharpness. Therefore, the same level of sharpness on the whole as the case of only grinding can be secured.

Furthermore, the knife according to the present invention is mainly used for cutting by thrusting the blade tip into an object, such as sclera, to a predetermined depth (axial direction), and moving orthogonally to the axis while maintaining it around that depth. Therefore, since the sharpness of the blade tip only when the knife is first thrust into tissue affects the technician's perception of the sharpness during use, and it is then cut by the edge lower (handle side) than the rounded portion of the blade tip being pulled back, the technician's perception of sharpness during use is not affected even if the blade tip is rounded.

Yet further, since the knife according to the present invention is often used for cutting by inserting approximately 1 to 2 mm from the front end and pulling back, unlike knife having the precisely formed maximal blade width for forming an incision into which an intraocular lens is to be inserted where the latter knife is not according to the present invention and is used to be moved along the axis and in the direction parallel to the cutting portion and to be inserted into a tissue, the angle of the cutting blade does not affect sharpness as is, and the sharpness is clearly perceived even if the angle of the cutting blade and the knife for forming an incision in order to insert the intraocular lens are the same.

The knife for forming an incision in order to insert the intraocular lens is not according to the present invention because formation of the knife, through only grinding, used for inserting the intraocular lens allows easy control of dimensions of the maximum blade width, and precise formation.

Yet even further, the configuration having the cutting blade gradually bending in a centrally protruding form makes a smaller angle of gradient of the edge to the tissue than the conventional knife which doesn't have a bent edge but have a straight edge while the angle of gradient of the knife handle to the tissue is large. As a result, sharpness is improved, thereby making a user-friendly knife. Yet even further, the configuration having the slanted surface formed by a wrapping film makes the slanted surface gradually bending in a centrally protruding form, and both end portions (ends on the front end side and the shank side) of the slanted surface are convex rounded surfaces, thereby increasing the angle of the slanted surface. Through administration of electrolytic polishing and chemical polishing, a cutting blade is molded exactly on the central protruding form, and both end portions with large angles are polished a little while the central portion with a small angle is polished a lot, thereby allowing formation of overall uniform cutting blade angles, and improving usability.

### [BRIEF DESCRIPTION OF THE DRAWINGS]

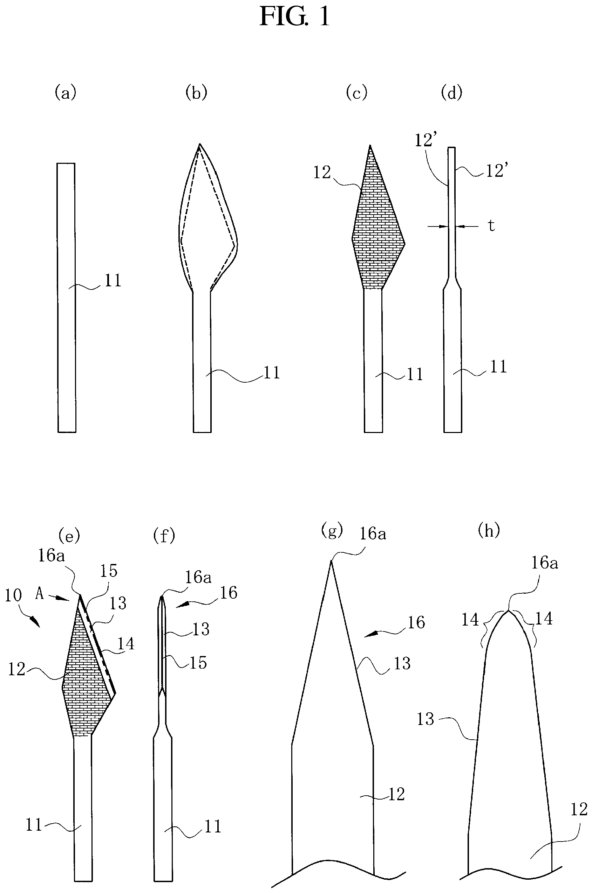

FIGS. 1(a) to 1(f) are diagrams describing a manufacturing method for a straight knife according to the present invention, and FIGS. 1(g) and 1(h) are enlarged views of a blade tip portion viewed from A of 1(e), where FIG. 1(g) illustrates a view before electrolytic polishing and FIG. 1(h) illustrates a view after electrolytic polishing;

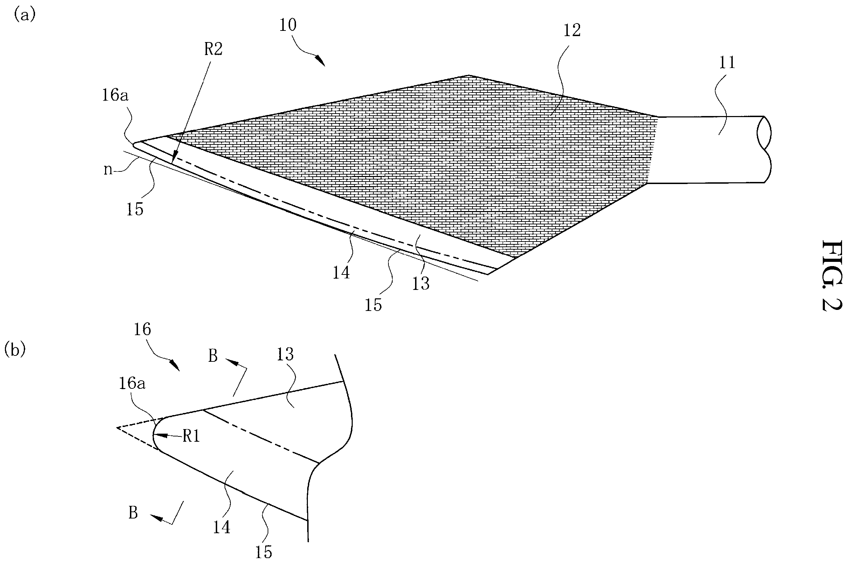

FIG. 2(a) is a top view of the straight knife, and FIG. 2(b) is an enlarged view of the blade tip portion;

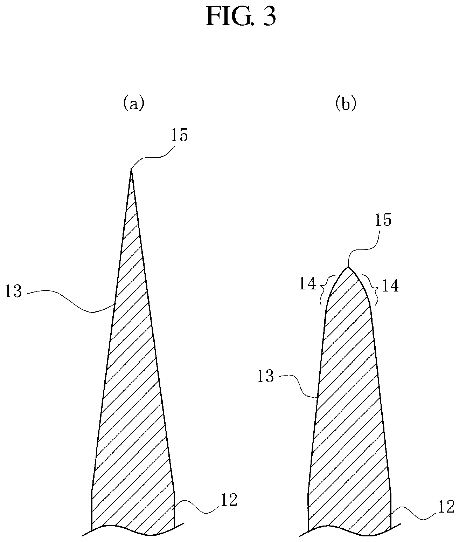

FIGS. 3 are cross sections cut along a line B-B of FIG. 2(b), where FIG. 3(a) illustrates a view before electrolytic polishing and FIG. 3(b) illustrates a view after electrolytic polishing;

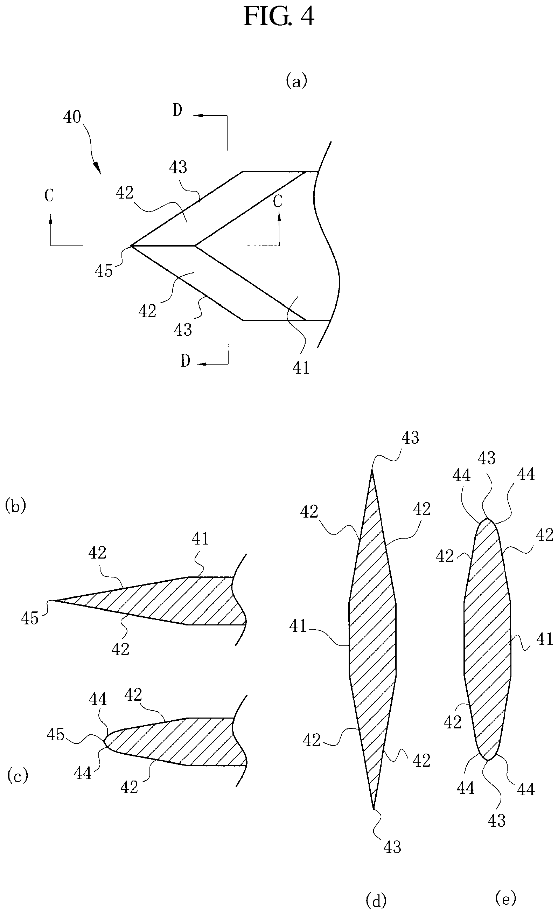

FIGS. 4 are diagrams of an LRI knife for astigmatism correction, where FIG. 4(a) is a top view, FIG. 4(b) is a cross section cut along a line C-C of FIG. 4(a) illustrating the state before electrolytic polishing, and FIG. 4(c) is a view illustrating the state after electrolytic polishing; FIG. 4(d) is a cross section cut along a line D-D of FIG. 4(a) illustrating the state before electrolytic polishing, and FIG. 4(e) is a view illustrating the state after electrolytic polishing; and
FIGS. 5 are diagrams illustrating a conventional straight knife, where FIG. 5(a) is a top view, FIG. 5(b) is a cross section of a cutting blade cut along a line E-E of FIG. 5(a), FIG. 5(c) is an enlarged view of a blade tip portion of the knife viewed from F of FIG. 5(a), and FIG. 5(d) is a diagram illustrating the blade tip portion in a bent state.

### [DESCRIPTION OF EMBODIMENTS]

An embodiment of the present invention is described while referencing the attached drawings.

FIGS. 1(a) to 1(f) are diagrams describing a manufacturing method for a straight knife 10 according to the present invention. To begin with, a raw, round bar 11 is cut at a predetermined length shown in FIG. 1(a). This is made from an austenitic stainless steel round bar having a fibrous crystalline structure. Then, as shown in FIG. 1(b), the front end side is flattened by a press, and excessive portions indicated by dotted lines are cut in a subsequent pressing step so as to have a planar pointed end, thereby forming an approximately rhombus cutting portion 12 viewed in the top view. As shown in FIGS. 1(c) and 1(d), both flattened surfaces 12' are polished and made into smooth surfaces and made into a predetermined thickness t. The flattened surfaces 12' of the cutting portion 12 are then made into irregular reflection surfaces through blasting. Making irregular reflection surfaces keeps the knife from shining and making it difficult to see an affected area when used under a microscope. Next, as illustrated in FIG. 1(e), a slanted surface 13 is formed on the respective flattened surfaces 12' through grinding, a front end portion of the slanted surface 13 is made into a cutting blade, and an edge 15 of the cutting blade is sharpened to a point. The slanted surfaces 13 are formed extending to the vicinity of the largest width of the cutting portion 12.

The above description is nearly the same as the manufacturing method of the conventional straight knife. The present invention is characterized by forming a cutting blade 14 through electrolytic polishing instead of grinding.

FIG. 1(g) is an enlarged view of a blade tip portion 16 viewed from A of FIG. 1(e), where FIG. 1(g) illustrates a view before electrolytic polishing and FIG. 1(h) illustrates a view after electrolytic polishing. The slanted surface 13 is formed through grinding using a grinding stone, has grooves orthogonal to the edge 15 formed across the entirety, and multiple burrs are generated on the slanted surface 13 and the edge 15.

With the present invention, once the slanted surfaces 13 are formed through grinding using a grinding stone, electrolytic polishing is carried out. Electrolytic polishing can be carried out using a typical method, such as a method of using phosphoric acid fluid as an electrolyte, soaking an entire cutting portion of a straight knife in an electrolytic solution, connecting the cutting portion to a positive electrode, connecting an electrolytic bath to a negative electrode, and electrify. The metal of the straight knife is eluted into the electrolytic solution, and is thereby polished. At this time, the metal at the pointed portion of the work is eluted first.

With electrolytic polishing, the burrs on the slanted surface 13 and the edge 15 are eluted into the electrolytic solution, thereby burr removal is carried out by priority. Typical electrolytic polishing is completed at this stage; however, with the present invention, electrolytic polishing is further continued.

Once the burrs are removed and electrolytic polishing is continued, grooves resulting from polishing are reduced, and the slanted surface 13 is melted so as to form the cutting blade 14 having a convex curved surface on an end of the slanted surface 13. The tip end of the cutting blade 14 is the edge 15.

FIG. 2(a) is an enlarged top view of the cutting portion 12 of the straight knife 10, and FIG. 2(b) is an enlarged view of the blade tip portion, where both FIGS. 2(a) and 2(b) illustrate the state after electrolytic polishing. A blade tip 16a has a dotted-line portion melted into a circular arc surface having radius R1. Carrying out electrolytic polishing up to this state after burr removal thickens the blade tip 16a, increasing its strength. Increase in strength allows prevention of bending of the blade tip portion 16 as shown in FIG. 5(d). Moreover, while the edge 15 of the cutting blade 14 is almost straight, it has a nearly circular form, gradually curving like the curvature of a Japanese sword. A straight line n shown in FIG. 2(a) is tangent to the edge 15.

Even the blade tip 16a of the straight knife 10 using the conventional martensitic stainless steel is not pointed but shows a certain amount of roundness when viewed from a microscope. Conventionally, this roundness has less than an R of 0.1 mm at the blade tip 16a; however, R1 of the blade tip according to the present invention is 0.3 to 0.7 mm, which is larger than the conventional radius. This is because R1 of less than 0.3 mm makes it thinner, thereby making it easier to be bent. If R1 is greater than 0.7 mm, a desired sharpness cannot be attained.

Furthermore, while radius R2 of the front end portion of the edge 15 is conventionally straight, with the present invention, polishing is continued after the burrs are removed, thereby becoming a curved line in nearly a circular arc form with R2 of 100 to 500 mm as illustrated in the drawing having the centrally protruding area. The centrally protruding curvature at R2 is also generated through electrolytic polishing; however, such a curvature allows improvement in sharpness of the knife. Particularly, since a portion approximately 1mm from the blade tip 16a is used with the straight knife 10, providing curvature to that portion can improve sharpness of the blade tip portion 16. Note that, as illustrated in FIG. 2(a), curvature may be provided near the widest portion of the cutting portion 12. If electrolytic polishing or chemical polishing is given to the whole cutting blade 14, curvature is formed near the widest portion. The reason for keeping R2 from becoming less than 100 mm is that when it is less than 100 mm, too much is ground, and sharpness decreases. Meanwhile, since cross-sectional angle of the blade (blade angle) when cutting by pulling looks smaller, the upper limit of R2 should be set to 500 mm or less. If it exceeds 500 mm, there is hardly any influence of reducing the cross-sectional angle of the blade.

By carrying out grinding for forming the slanted surface 13 using a wrapping film, the cutting portion 12 may be curved due to processing pressure during the grinding, making it easy to form a protruding curve indicated by R2 in FIG. 2. Moreover, the front end side and the shank side of the slanted surface 13 are curved convexly, and angle of gradient of the slanted surface 13 increases. Through administration of the same electrolytic polishing and chemical polishing as described above in this state, the cutting blade 14 is molded exactly on the centrally protruding form, and both end portions with large angles are polished a little while the central portion with a small angle is polished a lot, thereby allowing formation of overall uniform cutting blade angles.

While the blade tip 16a in FIG. 2(b) is a circular arc, it may actually have some irregularities, not being such a clean circular arc. However, 'become round' according to the present invention includes various rounded surfaces aside from the circular arc surface such as an elliptic surface, a paraboloidal surface, and a convex rounded surface with some irregularities.

The blade tip that is conventionally pointed as indicated by a dotted line in FIG. 2(b) is rounded as the blade tip 16a indicated by a solid line, however, sharpness is improved by making the convexly curved surface small as described above and eliminating the burrs, where sharpness on the whole is on par with that before electrolytic polishing. On the other hand, with the conventional pointed blade tip 16a, while the blade tip portion 16 is easily bent when cutting open a cornea or a sclera as described in FIG. 5(d), if the blade tip portion 16 at the front end of the cutting blades 14 constituted by convex rounded surfaces is used, cutting open without bending can be performed reliably and easily.

FIG. 3(a) is a diagram corresponding to FIG. 5(b) and is a cross section cut along a line B-B of FIG. 2(b). This illustrates the slanted surface 13 formed by grinding and the edge 15 of the cutting blades 14. The cutting blades 14 constituted by convex rounded surfaces are formed between the slanted surface 13 and the edge 15 through electrolytic polishing or chemical polishing. The cutting blades 14 have approximately circular arc surfaces and a central axis along the length of the slanted surface 13. Use of such a configuration allows easier penetration of the cutting blades 14 into bodily tissue and improvement in sharpness.

A knife having the slanted surface 13 and the cutting blades 14 formed symmetrically on either side of the cutting portion 12 has been described above; however, this also applies for a knife having the slanted surface 13 and the cutting blade 14 formed only one side of the cutting portion 12. Moreover, while a straight knife has been described as an example, this is applicable to other medical knives such as an LRI knife for astigmatism correction, for example.

FIGS. 4 are diagrams of an LRI knife for astigmatism correction, where FIG. 4(a) is a top view, FIG. 4(b) is a cross section cut along a line C-C of FIG. 4(a) illustrating the state before electrolytic polishing, and FIG. 4(c) is a view illustrating the state after electrolytic polishing. FIG. 4(d) is a cross section cut along a line D-D of FIG. 4(a) illustrating the state before electrolytic polishing, and FIG. 4(e) is a view illustrating the state after electrolytic polishing.

An LRI knife 40 has a cutting portion 41 having a pointed, almost 90 degree V-shaped front end, and slanted surfaces 42 formed on two sides constituting the front end V shape. Edges of the slanted surfaces 42 are edges 43 and 43. A blade tip 45 is at the intersecting point of the edges 43 and 43.

If the slanted surfaces 42 and the edges 43 are formed at the cutting portion 41 through grinding, the LRI knife 40 has cross-sectional shapes as illustrated in FIGS. 4(b) and 4(d), and if electrolytic polishing is carried out after burr removal according to the present invention, it has cross-sectional shapes as in FIGS. 4(c) and 4(e).

That is, cutting blades 44 constituted by convex rounded surfaces are formed on the front end side of the slanted surfaces 42, and the edges 43 are formed on front ends thereof. However, a blade tip 45 in the top view of FIG. 4(a) is not rounded as the straight knife 10 illustrated in FIG. 2(b). This is because the LRI knife 40 is thick from the start, the V-shaped angle is large, and if the cutting blades 44 constituted by convex rounded surfaces are formed, the problem of bending does not occur without rounding the blade tip 45. Moreover, the blade tip 45 may be rounded in the top view of FIG. 2(b).

Tables 1 and 2 are examples comparing sharpness of the straight knife 10 according to the present invention and a conventional straight knife. Table 1 shows the results from examining sharpness on ten specimens 1 to 10 using the straight knife of the present invention. Table 2 shows the results from examining sharpness on two specimens 11 and 12 using a conventional straight knife made of martensitic stainless steel. Both the straight knife according to the present invention and the conventional straight knife have a knife angle of 15 degrees, board thickness (value of t) of 0.11 mm, and the same shape. Testing has been conducted by measuring pierce force (unit of millinewtons 'mN') when the straight knife is pierced through a 0.45 mm-thick membrane of imitation leather (Porvair) having a hardness close to that of the cornea or sclera.

**[Table 1]**

| Working Example | | | | |
|---|---|---|---|---|
| Specimen Number | 1st | 2nd | 3rd | Average |
| | mN | mN | mN | mN |
| 1 | 237.0 | 297.0 | 383.0 | 305.7 |
| 2 | 269.0 | 338.0 | 396.0 | 334.3 |
| 3 | 248.0 | 319.0 | 392.0 | 319.7 |
| 4 | 246.0 | 293.0 | 334.0 | 291.0 |
| 5 | 192.0 | 280.0 | 313.0 | 261.7 |
| 6 | 241.0 | 349.0 | 383.0 | 324.3 |
| 7 | 227.0 | 315.0 | 414.0 | 318.7 |
| 8 | 246.0 | 315.0 | 379.0 | 313.3 |
| 9 | 255.0 | 310.0 | 351.0 | 305.3 |
| 10 | 295.0 | 349.0 | 383.0 | 342.3 |
| min. | 192.0 | 280.0 | 313.0 | 261.7 |
| max. | 295.0 | 349.0 | 414.0 | 342.3 |
| Average | 245.6 | 316.5 | 372.8 | 311.6 |

**[Table 2]**

| Conventional Example | | | | |
|---|---|---|---|---|
| Specimen Number | 1st | 2nd | 3rd | Average |
| | mN | mN | mN | mN |
| 11 | 333.0 | 393.0 | 428.0 | 384.7 |
| 12 | 293.0 | 345.0 | 393.0 | 343.7 |
| min. | 293.0 | 345.0 | 393.0 | 343.7 |
| max. | 333.0 | 393.0 | 428.0 | 384.7 |
| Average | 313.0 | 369.0 | 410.5 | 364.2 |

Table 1 shows results of a pierce test conducted three times for each of ten specimens using the straight knife according to the present invention. As a result, the blade tip portion has pierced through all of the specimens without even a single bent one. In addition, pierce resistance, although slightly, has improved more than the conventional straight knife made of martensitic stainless steel.

This shows that according to the present invention, a straight knife can be manufactured from austenitic stainless steel, with sharpness that bears comparison with the conventional knife.

Note that electrolytic polishing has been carried out in the above working examples, but chemical polishing is also possible. However, since roundness of the blade tip 16a of the cutting blades 14 shown in FIG. 1(g) is small, the blade tip 16a with such a small R cannot be formed by grinding. This roundness can be formed only by electrolytic polishing or chemical polishing.

### [Explanation of References]

10: straight knife
11: round bar
12: cutting portion
13: slanted surface
14: cutting blade
15: cutting blade edge
16: blade tip portion
16a: blade tip
17: curved surface

## Claims

1. A medical knife (10), comprising
a planar cutting portion (12),
a slanted surface (13) formed along the periphery of at least a part of the cutting portion (12) and a sharpened edge (15),
**characterised in that**
the slanted surface is electrolytically polished or chemically polished so as to form a cutting blade (14) constituted by a convex rounded surface between the slanted surface (13) and the edge (15).

2. The medical knife of Claim 1, wherein the cutting blade (14) is gradually curved in a direction of the cutting edge (15).

3. The medical knife of either Claim 1 or Claim 2, wherein the cutting blade (14) is formed on two sides of the cutting portion (12) adjacent to each other.

## Patentansprüche

1. Medizinisches Messer (10), umfassend:
einen planaren Schneidabschnitt (12),
eine schräge Fläche (13), die entlang des Umfangs mindestens eines Teils des Schneidabschnitts (12) und einer geschärften Kante (15) ausgebildet ist,
**dadurch gekennzeichnet, dass**
die schräge Fläche elektrolytisch poliert wird oder chemisch poliert wird, um eine Schneidklinge (14) zu bilden, die von einer konvexen, gerundeten Fläche zwischen der schrägen Fläche (13) und der Kante (15) gebildet ist.

2. Medizinisches Messer nach Anspruch 1, wobei die Schneidklinge (14) in Richtung der Schneidkante (15) allmählich gekrümmt ist.

3. Medizinisches Messer nach Anspruch 1 oder Anspruch 2, wobei die Schneidklinge (14) auf zwei Seiten des Schneidabschnitts (12) nebeneinander ausgebildet ist.

## Revendications

1. Couteau médical (10) comprenant :
une partie de coupe plane (12),
une surface inclinée (13) formée le long de la périphérie d'au moins une partie de la partie de coupe (12) et d'un bord affûté (15),
**caractérisé en ce que** :
la surface inclinée est polie électrolytiquement ou est polie chimiquement de manière à former une lame de coupe (14) constituée d'une surface arrondie convexe entre la surface inclinée (13) et le bord (15).

2. Couteau médical selon la revendication 1, dans lequel la lame de coupe (14) est progressivement incurvée dans la direction du bord de coupe (15).

3. Couteau médical selon la revendication 1 ou la revendication 2, dans lequel la lame de coupe (14) est formée des deux côtés de la partie de coupe (12) adjacents l'un à l'autre.
